# EUROPEAN PATENT SPECIFICATION

(11) **EP 2 391 366 B1**
(45) Date of publication and mention of the grant of the patent: **28.11.2012**
(21) Application number: 10703564.4
(22) Date of filing: 28.01.2010
(51) Int. Cl.: A61K 31/4439

(54) **SUBSTITUTED BENZIMIDAZOLES FOR THE TREATMENT OF ASTROCYTOMAS**
SUBSTITUIERTE BENZIMIDAZOLE ZUR BEHANDLUNG VON ASTROZYTOMEN
BENZIMIDAZOLES SUBSTITUÉS DESTINÉS AU TRAITEMENT D'ASTROCYTOMES

(30) Priority: 29.01.2009 US 148117 P
(43) Date of publication of application: 07.12.2011
(73) Proprietor: Novartis AG, 4056 Basel (CH)
(72) Inventor: STUART, Darrin, East Hanover, NJ 07936 (US)
(74) Representative: Guder, André
(86) International application number: PCT/US2010/022332
(87) International publication number: WO 2010/088335

(56) References cited:
- US-A1- 2004 087 626

## Description

The present invention relates to the use of SUBSTITUTED BENZIMIDAZOLES for the treatment of, and preparation of a drug for the treatment of astrocytomas.

### Background of the Invention

Astrocytomas are primary central nervous system tumors that arise primarily in and rarely spread away from the CNS parenchyma contained within the cranial vault. Astrocytomas "are CNS neoplasms in which the predominant cell type is derived from an astrocyte and account for roughly 75% of neuroepithelial tumors. In 1993 the World Health Organization (WHO) established a four-tiered histologic grading guideline for astrocytomas in an effort to eliminate confusion regarding diagnoses. These include Low-Grade (I and II) astrocytomas and more aggressive forms such as Anaplastic astrocytoma (Grade III) and Glioblastoma multiforme (Grade IV).

World Health Organization (WHO) grade 1 astrocytomas including pediatric astrocytomas (Pilomyxoid astrocytoma, pilocytic astrocytomas, pleomorphic xanthoastrocytomas, subependymal giant cell astrocytomas, and subependymomas) are uncommon tumors which can often be cured by surgically removing the tumor (resection). Even if the surgeon is not able to remove the entire tumor, it may remain inactive or be successfully treated with radiation.

Grade II tumors are defined as being infiltrative gliomas - the tumor cells penetrate into the surrounding normal brain, making a surgical cure more difficult.

Most patients with grade II glioma (oligodendrogliomas, astrocytomas, mixed oligoastrocytomas) are young people who often present with seizures. The median survival varies with the cell type of the tumor. People with oligodendrogliomas have better a prognosis than those with mixed oligoastrocytomas who have better a prognosis than someone with an astrocytoma. Other factors which influence survival include age (younger the better) and performance status (ability to perform tasks of daily living). Due to the infiltrative nature of these tumors, recurrences are relatively common. Depending on the patient, radiation or chemotherapy after surgery is an option.

Most grade II gliomas eventually evolve into more aggressive tumors (grade III or IV) and cannot be cured by surgery and radiation therapy. A practical approach is to remove as much of the abnormal tissue as possible without causing neurologic injury. Research has shown that beginning radiation therapy immediately after diagnosis delays recurrence compared to beginning radiation when there is evidence of tumor growth.

Patients with anaplastic astrocytoma (Grade III) often present with seizures, neurologic deficits, headaches, or changes in mental status. The standard initial treatment is to remove as much of the tumor as possible without worsening neurologic deficits. Radiation therapy has been shown to prolong survival and is a standard component of treatment. In general, median survival ranges from two to three years. There is no proven benefit to adjuvant chemotherapy (supplementing other treatments) for this kind of tumor. Although temozolomide is effective for treating recurrent anaplastic astrocytoma, its role as an adjuvant to radiation therapy has not tested.

Glioblastoma multiforme (Grade IV) is the most common and most malignant primary brain tumor.

The low grade astrocytomas (I & II) are among the least common of all reported brain tumors, less than 6%, while the highest grade (IV), also known as glioblastoma multiforme (GBM), is the most common primary CNS malignancy and second most frequent brain tumor. Despite the comparatively low incidence of astrocytomas to other human cancers, the higher grades (III & IV) represent disparate mortality rates. Median survival of GBM victims who forgo treatment is approximately 90 days, and even with aggressive surgical, radio- and chemo- therapies is only extended to about twelve months, while long term survival (at least five years) falls under 3%.

A Computed Tomography (CT) or Magnetic Resonance imaging (MRI) scan is necessary to characterize the extent of these tumors (size, location, consistency). CT will usually show distortion of third and lateral ventricles with displacement of anterior and middle cerebral arteries. Histologic analysis is necessary for grading diagnosis.

In the first stage of diagnosis the doctor will take a history of symptoms and perform a basic neurological exam, including an eye exam and tests of vision, balance, coordination and mental status. The doctor will then require a computerized tomography (CT) scan and magnetic resonance imaging (MRI) of the patient's brain. During a CT scan, x rays of the patient's brain are taken from many different directions; these are combined by a computer, producing a cross-sectional image of the brain. For an MRI, the patient relaxes in a tunnel-like instrument while the brain is subjected to changes of magnetic field. An image is produced based on the behavior of the brain's water molecules in response to the magnetic fields. A special dye may be injected into a vein before these scans to provide contrast and make tumors easier to identify.

If a tumor is found it will be necessary for a neurosurgeon to perform a biopsy on it. This simply involves the removal of a small amount of tumor tissue, which is then sent to a neuropathologist for examination and staging. The biopsy may take place before surgical removal of the tumor or the sample may be taken during surgery. Staging of the tumor sample is a method of classification that helps the doctor to determine the severity of the astrocytoma and to decide on the best treatment options. The neuropathologist stages the tumor by looking for atypical cells, the growth of new blood vessels, and for indicators of cell division called mitotic figures.

For low grade astrocytomas, removal of the tumor will generally allow functional survival for many years. In some reports, the 5 year survival has been over 90% with well resected tumors. Indeed, broad intervention of low grade conditions is a contested matter. In particular, pilocytic astrocytomas are commonly indolent bodies that may permit normal neurologic function. However, left unattended these tumors may eventually undergo neoplastic transformation. To date, complete resection of high grade astrocytomas is impossible because of the diffuse infiltration of tumor cells into normal parenchyma. Thus, high grade astrocytomas inevitably recur after initial surgery/therapy and are usually treated similarly as the initial tumor. Despite decades of therapeutic research, curative intervention is still nonexistent for high grade astrocytomas; patient care ultimately focuses on palliative management.

The Ras family of proto-oncogenes (N-Ras, K-Ras ,and H-Ras) and B-Raf, which encodes a Ras-regulated serine/threonine kinase with oncogenic properties and are essential components of the Ras/mitogen activated protein kinases (MAPK) signaling module as described in US Patent application No. 11/513,959, serve as signal transduction mediators promoting cell growth, differentiation, and survival signals. Activated Ras exists in a GTP-bound state, and inactivation occurs upon hydrolysis of GTP to GDP. Ras mutations are associated with several human malignancies and result in a decreased rate of GTP hydrolysis, leading to sustained activation.

Therapeutic interventions targeting these downstream signaling pathways represent a potential approach to treating astrocytomas. See Knobbe C., et al. Mutation analysis of the Ras pathway genes NRAS, HRAS, KRAS and BRAF in glioblastomas, Acta Neuropathol (2004) 108:467-470; Pfister S., et al. BRAF gene duplication constitutes a mechanism of MAPK pathway activation in low-grade astrocytomas, The Journal of Clinical Investigation (2008), Volume 118, No. 5: 1739-1749; Karajannis M., et al. Treatment of Pediatric Brain Tumors, J. Cell Physiol. 217: 584-589, 2008; Jones D., et al. Tandem Duplication Producing a Novel Oncogenic BRAF Fusion Gene Defines the Majority of Pilocyctic Aastrocyomas, Cancer Res 2008; 68: (21). November 1, 2008.

Benzamidazoles as described in US Patent No. 7,071,216 and US Patent application No. 11/513,959 are small molecule inhibitors of Raf kinase that has been shown to preferentially inhibit the Raf/MEK/ERK signaling pathway in tumor cells which express mutant, activated forms of Ras or B-Raf.

As an inhibitor of Raf/MEK/ERK signaling pathway, Benzimidazole derivatives have the potential to be of benefit in the treatment of astrocytomas.

### Brief description of the drawings

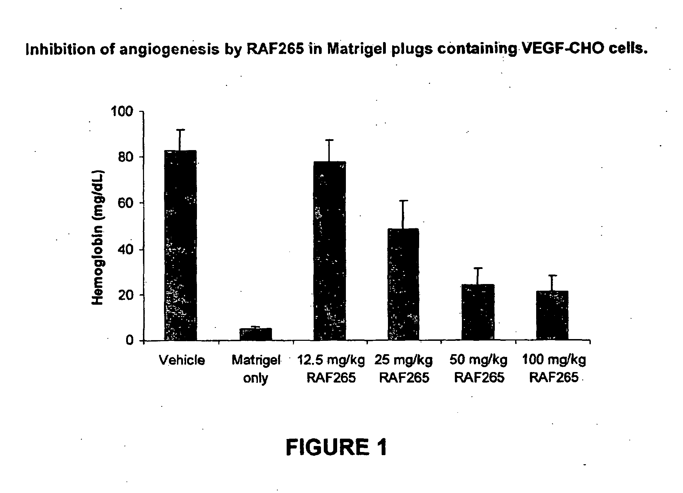

As shown in Figure 1, VEGF-CHO cells depicting induced angiogenesis.

### Summary of the Invention

The present invention relates to the use of benzimidazoles of formula (I), hereinafter "BENZIMIDAZOLE DERIVATIVES"). 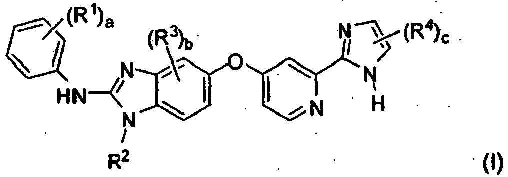 wherein,
each R¹ is independently selected from hydroxy, halo, C₁₋₆ alkyl, C₁₋₆ alkoxy, (C₁₋₆ alkyl)sulfanyl, (C₁₋₆ alkyl)sulfonyl, cycloalkyl, heterocycloalkyl, phenyl and heteroaryl;
R² is C₁₋₆ alkyl or halo(C₁₋₆ alkyl);
each R³ is independently selected from halo, C₁₋₆ alkyl, and C₁₋₆ alkoxy;
each R⁴ is independently selected from hydroxy, C₁₋₆ alkyl, C₁₋₆ alkoxy, halo, carboxyl, (C₁₋₆ alkoxy)carbonyl, aminocarbonyl, C₁₋₆ alkylaminocarbonyl, carbonitrile, cycloalkyl, heterocycloalkyl, heterocycloalkylcarbonyl, phenyl and heteroaryl;
wherein R¹, R², R³, and R⁴ may be optionally substituted with one or more substituents independently selected from hydroxy, halo, C₁₋₆ alkyl, halo(C₁₋₆ alkyl), C₁₋₆ alkoxy and halo(C₁₋₆ alkoxy);
a is 1, 2, 3, 4 or 5;
b is 0, 1, 2 or 3; and
c is 1 or 2;
or a tautomer or stereoisomer, thereof or a pharmaceutically acceptable salt of the compound, tautomer, or stereoisomer for use in treating or preventing astrocytomas.

The general terms used hereinbefore and hereinafter preferably have within the context of this disclosure the following meanings, unless otherwise indicated.

In yet other aspects, the present invention provides methods for treating Raf related disorders in a human or animal subject in need of such treatment comprising administering to said subject an amount of a compound of formula (I), (II) or (III) effective to reduce or prevent tumor growth in the subject in combination with at least one additional agent for the treatment of cancer. A number of suitable anticancer agents to be used as combination therapeutics are contemplated for use in the methods of the present.invention. Indeed, the present invention contemplates, but is not limited to, administration of numerous anticancer agents, such as agents that induce apoptosis; polynucleotides, e.g., ribozymes; polypeptides, e.g., enzymes; drugs; biological mimetics; alkaloids; alkylating agents; antitumor antibiotics; antimetabolites; hormones; platinum compounds; monoclonal antibodies conjugated with anticancer drugs, toxins, and/or radionuclides; biological response modifiers, e.g., interferons, e.g., IFN-a, etc.; and interleukins, e.g., IL-2, etc., adoptive immunotherapy agents; hematopoietic growth factors; agents that induce tumor cell differentiation, e.g., all-trans-retinoic acid, etc.; gene therapy reagents; antisense therapy reagents and nucleotides; tumor vaccines; inhibitors of angiogenesis, and the like. Numerous other examples of chemotherapeutic compounds and anticancer therapies suitable for coadministration with the disclosed compounds of formula (I), (II) or (III) are known to those skilled in the art.

In preferred embodiments, anticancer agents to be used in combination with compounds of the present invention comprise agents that induce or stimulate apoptosis. Agents that induce apoptosis include, but are not limited to, radiation; kinase inhibitors, e.g., epidermal growth factor receptor (EGFR) kinase inhibitor, vascular endothelial growth factor receptor (VEGFR) kinase inhibitor, fibroblast growth factor receptor (FGFR) kinase inhibitor, platelet-derived growth factor receptor (PGFR) I kinase inhibitor, and Bcr-Abl kinase inhibitors, such as STI-571, Gleevec, and Glivec; antisense molecules; antibodies, e.g., herceptin and rituxan; anti-estrogens, e.g., raloxifene and tamoxifen; anti-androgens, e.g., flutamide, bicalutamide, finasteride, amino-glutethamide, ketoconazole and corticosteroids; cyclooxygenase 2 (COX-2) inhibitors, e.g., celecoxib, meloxicam, NS-398 and non-steroidal antiinflammatory drugs (NSAIDs); and cancer chemotherapeutic drugs, e.g., irinotecan (camptosar), CPT-11, fludarabine (fludara), dacarbazine (DTIC), dexamethasone, mitoxantrone, mylotarg, VP-16, cisplatinum, 5-FU, doxrubicin, taxotere or taxol; cellular signaling molecules; ceramides and cytokines; and staurosprine, and the like.

In other aspects, the present invention provides pharmaceutical compositions comprising at least one compound or a pharmaceutically acceptable salt thereof of formula (I), (II) or (III) together with a pharmaceutically acceptable carrier suitable for administration to a human or animal subject, either alone or together with other anticancer agents.

"Raf inhibitor" is used herein to refer to a compound that exhibits an IC₅₀ with respect to Raf kinase activity of no more than about 100 µM and more typically not more than about 50 µM, as measured in the Raf/Mek Filtration Assay described generally hereinbelow. Preferred isoforms of Raf Kinase in which the compounds of the present invention will be shown to inhibit, include A-Raf, B-Raf, and C-Raf (Raf-1). "IC₅₀" is that concentration of inhibitor which reduces the activity of an enzyme, e.g., Raf kinase, to half-maximal level. Representative compounds of the present invention have been discovered to exhibit inhibitory activity against Raf. Compounds of the present invention preferably exhibit an IC₅₀ with respect to Raf of no more than about 10 µM, more preferably, no more than about 5 µM, even more preferably not more than about 1 µM, and most preferably, not more than about 200 nM, as measured in the Raf kinase assays described herein.

"Alkyl" refers to saturated hydrocarbyl groups that do not contain heteroatoms and includes straight chain alkyl groups, such as methyl, ethyl, propyl, butyl, pentyl, hexyl, heptyl, octyl, nonyl, decyl, undecyl, dodecyl and the like. Alkyl also includes branched chain isomers of straight chain alkyl groups, including but not limited to, the following which are provided by way of example: -CH(CH₃)₂, -CH(CH₃)(CH₂CH₃), -CH(CH₂CH₃)₂, -C(CH₃)₃, -C(CH₂CH₃)₃, -CH₂CH(CH₃)₂, -CH₂CH(CH₃)(CH₂CH₃), -CH₂CH(CH₂CH₃)₂, -CH₂C(CH₃)₃, -CH₂C(CH₂CH₃)₃, -CH(CH₃)-CH(CH₃)(CH₂CH₃), -CH₂CH₂CH(CH₃)₂, -CH₂CH₂CH(CH₃)(CH₂CH₃), -CH₂CH₂CH(CH₂CH₃)₂, -CH₂CH₂C(CH₃)₃, -CH₂CH₂C(CH₂CH₃)₃, -CH(CH₃)CH₂CH(CH₃)₂, -CH(CH₃)CH(CH₃)CH(CH₃)₂, -CH(CH₂CH₃)CH(CH₃)CH(CH₃)(CH₂CH₃) and others. Thus alkyl groups include primary alkyl groups, secondary alkyl groups and tertiary alkyl groups. The phrase "C₁₋₁₂ alkyl" refers to alkyl groups having from one to twelve carbon atoms. The phrase "C₁₋₆ alkyl" refers to alkyl groups having from one to six carbon atoms.

"Alkoxy" refers to RO-, wherein R is an alkyl group. The phrase "C₁₋₃ alkoxy", as used herein, refers to RO-, wherein R is a C₁₋₆ alkyl group. Representative examples of C₁₋₆ alkoxy groups include methoxy, ethoxy, t-butoxy and the like.

"(C₁₋₆ alkoxy)carbonyl" refers to ester -C(=O)-OR, wherein R is C₁₋₆ alkyl.

"Aminocarbonyl" refers herein to the group -C(O)-NH₂.

"C₁₋₆ alkylaminocarbonyl" refers to the group -C(O)-NRR', where R is C₁₋₆ alkyl and R' is selected from hydrogen and C₁₋₆ alkyl.

"Carbonyl" refers to the divalent group -C(O)-.

"Carboxyl" refers to -C(=O)-OH.

"Cyano", "carbonitrile" or "nitrile" refers to -CN.

"Carbonitrile(C₁₋₆ alkyl)" refers to C₁₋₆ alkyl substituted with -CN.

"Cycloalkyl" refers to a mono- or polycyclic alkyl substituent. Typical cycloalkyl groups have from 3 to 8 carbon ring atoms. Representative cycloalkyl groups include cyclopropyl, cyclobutyl, cyclopentyl, cyclohexyl, cycloheptyl and cyclooctyl.

"Halogen" or "halo" refers to chloro, bromo, fluoro and iodo groups.

"Halo(C₁₋₆ alkyl)" refers to a C₁₋₆ alkyl radical substituted with one or more halogen atoms, preferably one to five halogen atoms. A more preferred halo(C₁₋₆ alkyl) group is trifluoromethyl.

"Halo(C₁₋₆ alkyl)phenyl" refers to a phenyl group substituted with a halo(C₁₋₆ alkyl) group.

"Halo(C₁₋₆ alkoxy)" refers to an alkoxy radical substituted with one or more halogen atoms, preferably one to five halogen atoms. A more preferred halo(C₁₋₆ alkoxy) group is trifluoromethoxy.

"Halo(C₁₋₆ alkyl)sulfonyl" and "halo(C₁₋₆ alkyl)sulfanyl" refer to substitution of sulfonyl and sulfanyl groups with halo(C₁₋₆ alkyl) groups, wherein sulfonyl and sulfanyl are as defined herein.

"Heteroaryl" refers to an aromatic group having from 1 to 4 heteroatoms as ring atoms in an aromatic ring with the remainder of the ring atoms being carbon atoms. Suitable heteroatoms employed in compounds of the present invention are nitrogen, oxygen and sulfur, wherein the nitrogen and sulfur atoms may be optionally oxidized. Exemplary heteroaryl groups have 5 to 14 ring atoms and include, e.g., benzimidazolyl, benzothiazolyl, benzoxazolyl, diazapinyl, furanyl, pyrazinyl, pyrazolyl, pyridyl, pyridazinyl, pyrimidinyl, pyrroyl, oxazolyl, isoxazolyl, imidazolyl, indolyl, indazolyl, quinolinyl, isoquinolinyl, quinazolinyl, quinoxalinyl, thiazolyl, thienyl and triazolyl.

"Heterocycloalkyl" refers herein to cycloalkyl substituents that have from 1 to 5, and more typically from 1 to 2 heteroatoms in the ring structure. Suitable heteroatoms employed in compounds of the present invention are nitrogen, oxygen and sulfur, wherein the nitrogen and sulfur atoms may be optionally oxidized. Representative heterocycloalkyl moieties include, e.g., morpholino, piperazinyl, piperidinyl and the like.

"(C₁₋₆ alkyl)Heterocycloalkyl" refers to a heterocycloalkyl group substituted with a C₁₋₆ alkyl group.

"Heterocycloalkyl(C₁₋₆ alkyl)" refers to C₁₋₆ alkyl substituted with heterocycloalkyl.

"Heterocycloalkylcarbonyl" refers herein to the group -C(O)-R¹⁰, where R¹⁰ is heterocycloalkyl.

"(C₁₋₆alkyl)Heterocycloalkylcarbonyl" refers to.the group -C(O)-R¹¹, where R¹¹ is (C₁₋₆ alkyl)heterocycloalkyl.

"Hydroxy" refers to -OH.

"Hydroxy(C₁₋₆ alkyl)" refers to a C₁₋₆ alkyl group substituted with hydroxy.

"Hydroxy(C₁₋₆ alkylaminocarbonyl)" refers to a C₁₋₆ alkylaminocarbonyl group substituted with hydroxy.

"Sulfonyl" refers herein to the group -SO₂-.

"Sulfanyl" refers herein to the group -S-. "Alkylsulfonyl" refers to a substituted sulfonyl of the structure-SO₂R¹² in which R¹² is alkyl. "Alkylsulfanyl" refers to a substituted sulfanyl of the structure -SR¹² in which R¹² is alkyl. Alkylsuffonyl and alkylsulfanyl groups employed in compounds of the present invention include (C₁₋₆ alkyl)sulfonyl and (C₁₋₆ alkyl)sulfanyl. Thus, typical groups include, e.g., methylsulfonyl and methylsulfanyl (i.e., where R¹² is methyl), ethylsulfonyl, and ethylsulfanyl (i.e., where R¹² is ethyl), propylsulfonyl, and propylsulfanyl (i.e., where R¹² is propyl) and the like.

"Hydroxy protecting group" refers to protecting groups for an OH group. The term, as used herein, also refers to protection of the.OH group of an acid COOH. Suitable hydroxy protecting groups, as well as suitable conditions for protecting and deprotecting particular functional groups are well-known in the art. For example, numerous such protecting groups are described in T. W. Greene and P. G. M. Wuts, Protecting Groups in Organic Synthesis, Third Edition, Wiley, New York (1999). Such hydroxy protecting groups include C₁₋₆alkyl ethers, benzyl ethers, p-methoxybenzyl ethers, silyl ethers and the like.

"Optionally substituted" or "substituted" refers to the replacement of one or more hydrogen atoms with a monovalent or divalent radical.

When the substituted substituent includes a straight chain group, the substitution can occur either within the chain, e.g., 2-hydroxypropyl, 2-aminobutyl and the like; or at the chain terminus, e.g., 2-hydroxyethyl, 3-cyanopropyl and the like. Substituted substitutents can be straight chain, branched or cyclic arrangements of covalently bonded carbon or heteroatoms.

It is understood that the above definitions are not intended to include impermissible substitution patterns, e.g., methyl substituted with five fluoro groups or a halogen atom substituted with another halogen atom. Such impermissible substitution patterns are well-known to the skilled artisan.

Compounds within the scope of formula (I) and the process for their manufacture are disclosed in U.S. Patent No. 7,071,216, U.S. Patent Application No. 11/513,959 and U.S. Patent Application No. 11/513,745 which are hereby incorporated into the present application by reference. A preferred compound is 1-methyl-5-[2-(5-trifluoromethyl-1H-imidazol-2-yl)-pyridin-4-yloxy]-1H-benzoimidazol-2-yl)-(4-trifluoromethylphenyl)-amine and pharmacutially acceptable salts thereof of formula (II): 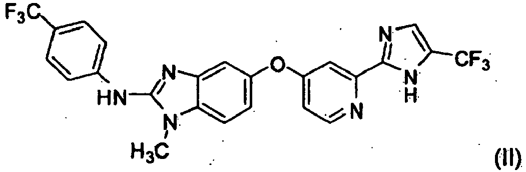 or a tautomer of the compound of formula (II) or a pharmaceutically acceptable salt of the tautomer having the formula (III):

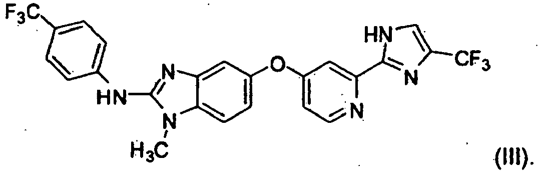

The structure of the active agents identified by code nos., generic or trade names may be taken from the actual edition of the standard compendium "The Merck Index" or from databases, e.g., Patents International, e.g., IMS World Publications.

It has now surprisingly been found that BENZIMIDAZOLE DERIVATIVES possess therapeutic properties, which render them useful to treat astrocytomas.

The present invention thus concerns the use of BENZIMIDAZOLE DERIVATIVES for the preparation of a drug for the treatment of astrocytomas.

The present invention more particularly concerns the use of BENZIMIDAZOLE DERIVATIVES for the preparation of a drug for the treatment of astrocytomas.

In another embodiment, the instant invention provides a method for treating astrocytomas comprising administering to a mammal in need of such treatment a therapeutically effective amount of BENZIMIDAZOLE DERIVATIVES, or pharmaceutically acceptable salts or prodrugs thereof.

Preferably the instant invention provides a method for treating mammals, especially humans, suffering from astrocytomas comprising administering to a mammal in need of such treatment an inhibiting amount of 1-methyl-5-[2-(5-trifluoromethyl-1H-imidazol-2-yl)-pyridin-4-yloxy]-1H-benzoimidazol-2-yl}-(4-trifluoro-methylphenyl)-amine (Compound (II)) or a pharmaceutically acceptable salt thereof.

In another embodiment, the instant invention relates to the use of BENZIMIDAZOLE DERIVATIVES for the preparation of a pharmaceutical composition for use in treating astrocytomas.

In the present description, the term "treatment" includes both prophylactic or preventative treatment, as well as curative or disease suppressive treatment, including treatment of patients at risk of contracting the disease or suspected to have contracted the disease, as well as ill patients. This term further includes the treatment for the delay of progression of the disease.

The term "curative", as used herein, means efficacy in treating ongoing episodes involving astrocytomas.

The term "prophylactic" means the prevention of the onset or recurrence of diseases involving astrocytomas.

The term "delay of progression", as used herein, means administration of the active compound to patients being in a pre-stage or in an early phase of the disease to be treated, in which patients, e.g., a pre-form of the corresponding disease is diagnosed or which patients are in a condition, e.g., during a medical treatment or a condition resulting from an accident, under which it is likely that a corresponding disease will develop.

This unforeseeable range of properties means that the use of BENZIMIDAZOLE DERIVATIVES are of particular interest for the manufacture of a medicament for the treatment of astrocytomas.

To demonstrate that BENZIMIDAZOLE DERIVATIVES are particularly suitable for the treatment of astrocytomas with good therapeutic margin and other advantages, clinical trials can be carried out in a manner known to the skilled person.

The precise dosage of BENZIMIDAZOLE DERIVATIVES to be employed for inhibiting and/or treating astrocytomas depends upon several factors including the host, the nature and the severity of the condition being treated, the mode of administration. The compound of formula (I) can be administered by any route including orally, parenterally, e.g., intraperitoneally, intravenously, intramuscularly, subcutaneously, intratumorally, or rectally, or enterally. Preferably, the compound of formula (I) is administered orally, preferably at a daily dosage of 1-300 mg/kg body weight or, for most larger primates, a daily dosage of 50-5000 mg, preferably 500-3000 mg.

Usually, a small dose is administered initially and the dosage is gradually increased until the optimal dosage for the host under treatment is determined. The upper limit of dosage is that imposed by side effects and can be determined by trial for the host being treated.

Compounds of formula (I) may be combined with one or more pharmaceutically acceptable carriers and, optionally, one or more other conventional pharmaceutical adjuvants and administered enterally, e.g., orally, in the form of tablets, capsules, caplets, etc. or parenterally, e.g., intraperitoneally or intravenously, in the form of sterile injectable solutions or suspensions. The enteral and parenteral compositions may be prepared by conventional means.

The BENZIMIDAZOLE DERIVATIVES can be used alone or combined with at least one other pharmaceutically active compound for use in these pathologies. Combination partners include antiproliferative compounds. Such antiproliferative compounds includes, but are not limited to aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (TEMODAL®); kinesin spindle protein inhibitors, such as SB715992 or SB743921 from GlaxoSmithkiine, or pentamidine/chlorpromazine from CombinatoRx; MEK inhibitors such as ARRY142886 from Array PioPharma, AZD6244 from AstraZeneca, PD181461 from Pfizer and leucovorin.

The term "aromatase inhibitor", as used herein, relates to a compound which inhibits the estrogen production, i.e., the conversion of the substrates androstenedione and testosterone to estrone and estradiol, respectively. The term includes, but is not limited to, steroids, especially atamestane, exemestane and formestane and, in particular; non-steroids, especially aminoglutethimide, roglethimide, pyridoglutethimide, trilostane, testolactone, ketokonazole, vorozole, fadrozole, anastrozole and letrozole. Exemestane can be administered, e.g., in the form as it is marketed, e.g., under the trademark AROMASIN. Formestane can be administered, e.g., in the form as it is marketed, e.g., under the trademark LENTARON. Fadrozole can be administered, e.g., in the form as it is marketed, e.g., under the trademark AFEMA. Anastrozole can be administered, e.g., in the form as it is marketed, e.g., under the trademark ARIMIDEX. Letrozole can be administered, e.g., in the form as it is marketed, e.g.. under the trademark FEMARA or FEMAR. Aminoglutethimide can be administered, e.g., in the form as it is marketed, e.g., under the trademark ORIMETEN. A combination of the invention comprising a chemotherapeutic agent which is an aromatase inhibitor is particularly useful for the treatment of hormone receptor positive tumors, e.g., breast tumors.

The term "antiestrogen", as used herein, relates to a compound which antagonizes the effect of estrogens at the estrogen receptor level. The term includes, but is not limited to, tamoxifen, fulvestrant, raloxifene and raloxifene hydrochloride. Tamoxifen can be administered, e.g., in the form as it is marketed, e.g., under the trademark NOLVADEX. Raloxifene hydrochloride can be administered, e.g., in the form as it is marketed, e.g., under the trademark EVISTA. Fulvestrant can be formulated as disclosed in U.S. Patent No. 4,659,516 or it can be administered, e.g., in the form as it is marketed, e.g., under the trademark FASLODEX. A combination of the invention comprising a chemotherapeutic agent which is an antiestrogen is particularly useful for the treatment of estrogen receptor positive tumors, e.g., breast tumors.

The term "anti-androgen"; as used herein, relates to any substance which is capable of inhibiting the biological effects of androgenic hormones and includes, but is not limited to, bicalutamide (CASODEX), which can be formulated, e.g., as disclosed in U.S. Patent No. 4,636,505.

The term "gonadorelin agonist", as used herein, includes, but is not limited to, abarelix, goserelin and goserelin acetate. Goserelin is disclosed in U.S. Patent No. 4,100,274 and can be administered, e.g., in the form as it is marketed, e.g., under the trademark ZOLADEX. Abarelix can be formulated, e.g., as disclosed in U.S. Patent No. 5,843,901.

The term "topoisomerase I inhibitor", as used herein, includes, but is not limited to, topotecan, gimatecan, irinotecan, camptothecian and its analogues, 9-nitrocamptothecin and the macromolecular camptothecin conjugate PNU-166148 (compound A1 in WO99/ 17804). Irinotecan can be administered, e.g., in the form as it is marketed, e.g., under the trademark CAMPTOSAR. Topotecan can be administered, e.g., in the form as it is marketed, e.g., under the trademark HYCAMTIN.

The term "topoisomerase II inhibitor", as used herein, includes, but is not limited to, the anthracyclines, such as doxorubicin (including liposomal formutation, e.g., CAELYX), daunorubicin, epirubicin, idarubicin and nemorubicin, the anthraquinones mitoxantrone and losoxantrone, and the podophillotoxines etoposide and teniposide. Etoposide can be administered, e.g., in the form as it is marketed, e.g., under the trademark ETOPOPHOS. Teniposide can be administered, e.g., in the form as it is marketed, e.g., under the trademark VM 26-BRISTOL. Doxorubicin can be administered, e.g., in the form as it is marketed, e.g., under the trademark ADRIBLASTIN or ADRIAMYCIN. Epirubicin can be administered, e.g., in the form as it is marketed, e.g., under the trademark FARMORUBICIN. Idarubicin can be administered, e.g., in the form as it is marketed, e.g., under the trademark ZAVEDOS. Mitoxantrone can be administered, e.g., in the form as it is marketed, e.g., under the trademark NOVANTRON.

The term "microtubule active agent" relates to microtubule stabilizing, microtubule destabilizing compounds and microtublin polymerization inhibitors including, but not limited to, taxanes, e.g., paclitaxel and docetaxel, vinca alkaloids, e.g., vinblastine, especially vinblastine sulfate, vincristine especially vincristine sulfate, and vinorelbine, discodermolides, cochicine and epothilones and derivatives thereof, e.g., epothilone B or D or derivatives thereof. Paclitaxel may be administered, e.g., in the form as it is marketed, e.g., TAXOL. Docetaxel can be administered, e.g., in the form as it is marketed, e.g., under the trademark TAXOTERE. Vinblastine sulfate can be administered, e.g., in the form as it is marketed, e.g., under the trademark VINBLASTIN R.P. Vincristine sulfate can be administered, e.g., in the form as it is marketed, e.g., under the trademark FARMISTIN. Discodermolide can be obtained, e.g., as disclosed in U.S. Patent No. 5,010,099. Also included are epothilone derivatives which are disclosed in WO 98/10121, U.S. Patent No. 6,194,181, WO 98/25929, WO 98/08849, WO 99/43653, WO 98/22461 and WO 00/31247. Especially preferred are epothilone A and/or B.

The term "alkylating agent", as used herein, includes, but is not limited to, cyclophosphamide, ifosfamide, melphalan or nitrosourea (BCNU or Gliadel). Cyclophosphamide can be administered, e.g., in the form as it is marketed, e.g., under the trademark CYCLOSTIN. Ifosfamide can be administered, e.g., in the form as it is marketed, e.g., under the trademark HOLOXAN.

The term "histone deacetylase inhibitors" or "HDAC inhibitors" rotates to compounds which inhibit the histone deacetylase and which possess antiproliferative activity. This includes compounds disclosed in WO 02/22577, especially N-hydroxy-3-[4-[[(2-hydroxyethyl)[2-(1H-indol-3-yl)ethyl]-amino]methyl]phenyl)-2E-2-propenamide, N-hydroxy-3-[4-[[[2-(2-methyl-1H-indol-3-yl)-ethyl]-amino]methyl]phenyl]-2E-2-propenamide and pharmaceutically acceptable salts thereof. It further especially includes Suberoylanilide hydroxamic acid (SAHA).

The term "antineoplastic antimetabolite" includes, but is not limited to, 5-fluorouracil or 5-FU, capecitabine, gemcitabine, DNA demethylating compounds, such as 5-azacytidine and decitabine, methotrexate and edatrexate, and folic acid antagonists, such as pemetrexed. Capecitabine can be administered, e.g., in the form as it is marketed, e.g., under the trademark XELODA. Gemcitabine can be administered, e.g., in the form as it is marketed, e.g., under the trademark GEMZAR.

The term "platin compound", as used herein, includes, but is not limited to, carboplatin, cisplatin, cisplatinum and oxaliplatin. Carboplatin can be administered, e.g., in the form as it is marketed, e.g., under the trademark CARBOPLAT. Oxaliplatin can be administered, e.g., in the form as it is marketed, e.g., under the trademark ELOXATIN.

The term "compounds targeting/decreasing a protein or lipid kinase activity; or a protein or lipid phosphatase activity; or further anti-angiogenic compounds", as used herein, includes, but is not limited to, protein tyrosine kinase and/or serine and/or threonine kinase inhibitors or lipid kinase inhibitors, e.g.,
a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g., a N-phenyl-2-pyrimidine-amine derivative, e.g., imatinib, SU101, SU6668 and GFB-111;
b) compounds targeting, decreasing or inhibiting the activity of the fibroblast growth factor-receptors (FGFR);
c) compounds targeting, decreasing or inhibiting the activity of the insulin-like growth factor receptor I (IGF-IR), such as compounds which target, decrease or inhibit the activity of IGF-IR, especially compounds which inhibit the kinase activity of TGF-I receptor, such as those compounds disclosed in WO 02/092599, or antibodies that target the extracellular domain of IGF-I receptor or its growth factors;
d) compounds targeting, decreasing or inhibiting the activity of the Trk receptor tyrosine kinase family, or ephrin B4. inhibitors;
e) compounds targeting, decreasing or inhibiting the activity of the Axl receptor tyrosine kinase family;
f) compounds targeting, decreasing or inhibiting the activity of the Ret receptor tyrosine kinase;
g) compounds targeting, decreasing or inhibiting the activity of the Kit/SCFR receptor tyrosine kinase, e.g., imatinib;
h) compounds targeting, decreasing or inhibiting the activity of the C-kit receptor tyrosine kinases - (part of the PDGFR family), such as compounds which target, decrease or inhibit the activity of the c-Kit receptor tyrosine kinase family, especially compounds which inhibit the c-Kit receptor, e.g., imatinib;
i) compounds targeting, decreasing or inhibiting the activity of members of the c-Abl family, their gene-fusion products (e.g., BCR-Abl kinase) and mutants, such as compounds which target decrease or inhibit the activity of c-Abl family members and their gene fusion products, e.g., a N-phenyl-2-pyrimidine-amine derivative, e.g., imatinib or nilotinib (AMN107); PD180970; AG957; NSC 680410; PD173955 from ParkeDavis; or dasatinib (BMS-354825);
j) compounds targeting, decreasing or inhibiting the activity of members of the protein kinase C (PKC) and Raf family of serine/threonine kinases, members of the MEK, SRC, JAK, FAK, PDK1, PKB/Akt, and Ras/MAPK family members, and/or members of the cyclin-dependent kinase family (CDK) and are especially those staurosporine derivatives disclosed in U.S. Patent No. 5,093,330, e.g., midostaurin; examples of further compounds include, e.g., UCN-01, safingol, BAY 43-9006, Bryostatin 1, Perifosine; limofosine; RO 318220 and RO 320432; GO 6976; Isis 3521; LY333531/LY379196; isochinoline compounds, such as those disclosed in WO 00/09495; FTIs; PD184352 or QAN697 (a P13K inhibitor) or AT7519 (CDK inhibitor);
k) compounds targeting, decreasing or inhibiting the activity of protein-tyrosine kinase inhibitors, such as compounds which target, decrease or inhibit the activity of protein-tyrosine kinase inhibitors include imatinib mesylate (GLEEVEC) or tyrphostin. A tyrphostin is preferably a low molecular weight (Mr < 1500) compound, or a pharmaceutically acceptable salt thereof, especially a compound selected from the benzylidenemalonitrite class or the S-arylbenzenemalonirile or bisubstrate quinoline class of compounds, more especially any compound selected from the group consisting of Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556, AG957 and adaphostin (4-{[(2,5-dihydroxyphenyl)methyl]amino}benzoic acid adamantyl ester; NSC 680410, adaphostin);
l) compounds targeting, decreasing or inhibiting the activity of the epidermal growth factor family of receptor tyrosine kinases (EGFR, ErbB2, ErbB3, ErbB4 as homo- or heterodimers) and their mutants, such as compounds which target, decrease or inhibit the activity of the epidermal growth factor receptor family are especially compounds, proteins or antibodies which inhibit members of the EGF receptor tyrosine kinase family, e.g., EGF receptor, ErbB2, ErbB3 and ErbB4 or bind to EGF or EGF related ligands, and are in particular those compounds, proteins or monoclonal antibodies generically and specifically disclosed in WO 97/02266, e.g., the compound of ex. 39, or in EP 0 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/30347, e.g., compound known as CP 358774, WO 96/33980, e.g., compound ZD 1839 and WO 95/03283, e.g., compound ZM105180; e.g. trastuzumab (Herceptin™), cetuximab (Erbitux™), Iressa, Tarceva, OSI-774, CI-1033, EKB-569, GW-2016, E1.1, E2.4, E2.5, E6.2, E6.4, E2.11, E6.3 or E7.6.3, and 7H-pyrrolo-[2,3-d]pyrimidine derivatives which are disclosed in WO 03/013541; and
m) compounds targeting, decreasing or inhibiting the activity of the c-Met receptor, such as compounds which target, decrease or inhibit the activity of c-Met, especially compounds which inhibit the kinase activity of c-Met receptor, or antibodies that target the extracellular domain of c-Met or bind to HGF.

Further anti-angiogenic compounds include compounds having another mechanism for their activity, e.g., unrelated to protein or lipid kinase inhibition, e.g., thalidomide (THALOMID) and TNP-470.

Compounds which target, decrease or inhibit the activity of a protein or lipid phosphatase are, e.g., inhibitors of phosphatase 1, phosphatase 2A, or CDC25, e.g., okadaic acid or a derivative thereof.

Compounds which induce cell differentiation processes are, e.g., retinoic acid, α-, γ- or δ-tocopherol or α-, γ- or δ-tocotrienol.

The term "cyclooxygenase inhibitor", as used herein, includes, but is not limited to, e.g., Cox-2 inhibitors, 5-alkyl substituted 2-arylaminophenylacetic acid and derivatives, such as celecoxib (CELEBREX), rofecoxib (VIOXX), etoricoxib, valdecoxib or a 5-alkyl-2-arylaminophenylacetic acid, e.g., 5-methyl-2-(2'-chloro-6'-fluoroanilino)phenyl acetic acid, lumiracoxib.

The term "bisphosphonates", as used herein, includes, but is not limited to, etridonic, clodronic, tiludronic, pamidronic, alendronic, ibandronic, risedronic and zoledronic acid. "Etridonic acid" can be administered, e.g., in the form as it is marketed, e.g., under the trademark DIDRONEL. "Clodronic acid" can be administered, e.g., in the form as it is marketed, e.g., under the trademark BONEFOS. "Tiludronic acid" can be administered, e.g., in the form as it is marketed, e.g., under the trademark SKELID. "Pamidronic acid" can be administered, e.g., in the form as it is marketed, e.g., under the trademark AREDIA™. "Alendronic acid" can be administered, e.g., in the form as it is marketed, e.g., under the trademark FOSAMAX. "Ibandronic acid" can be administered, e.g., in the form as it is marketed, e.g., under the trademark BONDRANAT. "Risedronic acid" can be administered, e.g., in the form as it is marketed, e.g., under the trademark ACTONEL. "Zoledronic acid" can be administered, e.g., in the form as it is marketed, e.g., under the trademark ZOMETA.

The term "mTOR inhibitors" relates to compounds which inhibit the mammalian target of rapamycin (mTOR) and which possess antiproliferative activity, such as sirolimus (Rapamune®), everolimus (Certican™), CCI-779 and ABT578.

The term "heparanase inhibitor", as used herein, refers to compounds which target, decrease or inhibit heparin sulfate degradation. The term includes, but is not limited to, PI-88.

The term "biological response modifier", as used herein, refers to a lymphokine or interferons, e.g., interferon γ.

The term "inhibitor of Ras oncogenic isoforms", e.g., H-Ras, K-Ras or N-Ras, as used herein, refers to compounds which target, decrease or inhibit the oncogenic activity of Ras e.g. a "farnesyl transferase inhibitor", e.g., L-744832, DK8G557 or R115777 (Zarnestra). The term "telomerase inhibitor", as used herein, refers to compounds which target, decrease or inhibit the activity of tetomerase. Compounds which target, decrease or inhibit the activity of telomerase are especially compounds which inhibit the telomerase receptor, e.g., telomestatin.

The term "methionine aminopeptidase inhibitor", as used herein, refers to compounds which target, decrease or inhibit the activity of methionine aminopeptidase. Compounds which target, decrease or inhibit the activity of methionine aminopeptidase are, e.g., bengamide or a derivative thereof.

The term "proteasome inhibitor", as used herein, refers to compounds which target, decrease or inhibit the activity of the proteasome. Compounds which target; decrease or inhibit the activity of the proteasome include, e.g., Bortezomid (Velcade™) and MLN 341.

The term "matrix metalloproteinase inhibitor" or ("MMP" inhibitor), as used herein, includes, but is not limited to, collagen peptidomimetic and nonpeptidomimetic inhibitors, tetracycline derivatives, e.g., hydroxamate peptidomimetic inhibitor batimastat and its orally bioavailable analogue marimastat (BB-2516), prinomastat (AG3340), metastat (NSC 683551) BMS-279251, BAY 12-9566, TAA211, MMI270B or AAJ996.

The term "compounds used in the treatment of hematologic malignancies", as used herein, includes, but is not limited to, FMS-like tyrosine kinase inhibitors, e.g., compounds targeting, decreasing or inhibiting the activity of FMS-like tyrosine kinase receptors (Flt-3R); interferon, 1-b-D-arabinofuransylcytosine (ara-c) and bisulfan; and ALK inhibitors, e.g., compounds which target, decrease or inhibit anaplastic lymphoma kinase.

Compounds which target, decrease or inhibit the activity of FMS-like tyrosine kinase receptors (Flt-3R) are especially compounds, proteins or antibodies which inhibit members of the Flt-3R receptor kinase family, e.g., PKC412, midostaurin, a staurosporine derivative, SU11248 and MLN518.

The term "HSP90 inhibitors", as used herein, includes, but is not limited to, compounds targeting, decreasing or inhibiting the intrinsic ATPase activity of HSP90; degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90, e.g., 17-allylamino,17-demethoxygeldanamycin (17AAG), a geldanamycin. derivative; other geldanamycin related compounds; radicicol and HDAC inhibitors.

The term "antiproliferative antibodies", as used herein, includes, but is-not limited to, trastuzumab (Herceptin™), Trastuzumab-DM1,erbitux, bevacizumab (Avastin™), rituximab (Rituxan®), PRO64553 (anti-CD40) and 2C4 antibody. By antibodies is meant, e.g., intact monoclonal antibodies, polyclonal antibodies, multispecific antibodies formed from at least 2 intact antibodies, and antibodies fragments so long as they exhibit the desired biological activity.

The term "antileukemic compounds" includes, e.g., Ara-C, a pyrimidine analog, which is the 2'-alpha-hydroxy ribose (arabinoside) derivative of deoxycytidine. Also included is the purine analog of hypoxanthine, 6-mercaptopurine (6-MP) and fludarabine phosphate.

Compounds which target, decrease or inhibit activity of histone deacetylase (HDAC) inhibitors, such as sodium butyrate and suberoylanilide hydroxamic acid (SAHA) inhibit the activity of the enzymes known as histone deacetylases. Specific HDAC inhibitors include MS275, SAHA, FK228 (formerly FR901228), Trichostatin A and compounds disclosed in U.S. Patent No. 6,552,065, in particular, N-hydroxy-3-[4-[[[2-(2-methyl-1H-indol-3-yl)-ethyl]-amino]methyl]phenyl]-2E-2-propenamide, or a pharmaceutically acceptable salt thereof and N-hydroxy-3-[4-[(2-hydroxyethyl){2-(1H-indol-3-yl)ethyl]-amino]methyl]phenyl]-2E-2-propenamide, or a pharmaceutically acceptable salt thereof, especially the lactate salt.

Somatostatin receptor antagonists as used herein refers to compounds which target, treat or inhibit the somatostatin receptor, such as octreotide, and SOM230.

Tumor cell damaging approaches refer to approaches, such as ionizing radiation. The term "ionizing radiation" referred to above and hereinafter means ionizing radiation that occurs as either electromagnetic rays, such as X-rays and gamma rays; or particles, such as alpha and beta particles. Ionizing radiation is provided in, but not limited to, radiation therapy and is known in the art. See Hellman, Principles of Radiation Therapy, Cancer, in Principles and Practice of Oncology, Devita et al., Eds., 4th Edition, Vol: 1, pp. 248-275 (1993)_{:}

The term EDG binders as used herein refers a class of immunosuppressants that modulates lymphocyte recirculation, such as FTY720.

The term ribonucleotide reductase inhibitors refers to pyrimidine or purine nucleoside analogs including, but not limited to, fludarabine and/or cytosine arabinoside (ara-C), 6-thioguanine, 5-fluorouracil, cladribine, 6-mercaptopurine (especially in combination with ara-C against ALL) and/or pentostatin. Ribonucleotide reductase inhibitors are especially hydroxyurea or 2-hydroxy-1H-isoindole-1,3-dione derivatives, such as PL-1, PL-2, PL-3, PL-4, PL-5, PL-6, PL-7 or PL-8 mentioned in Nandy et al., Acta Oncologica, Vol. 33, No. 8, pp. 953-961 (1994).

The term "S-adenosylmethionine decarboxylase inhibitors", as used herein, includes, but is not limited to, the compounds disclosed in U.S. Patent No. 5,461,076.

Also included are in particular those compounds, proteins or monoclonal antibodies of VEGF disclosed in WO. 98/35958, e.g., 1-(4-chloroanilino)-4-(4-pyridylmethyl)phthalazine or a pharmaceutically acceptable salt thereof, e.g., the succinate, or in WO 00/09495, WO 00/27820, WO 00/59509, WO 98/11223, WO 00/27819 and EP 0 769 947; those as described by Prewett et al, Cancer Res, Vol. 59, pp. 5209-5218 (1999); Yuan et al., Proc Natl Acad Sci U S A, Vol. 93, pp. 14765-14770 (1996); Zhu et al., Cancer Res, Vol. 58, pp. 3209-3214 (1998); and Mordenti et al., Toxicol Pathol, Vol. 27, No. 1. pp. 14-21 (1999); in WO 00/37502 and WO 94/10202; ANGIOSTATIN, described by O'Reilly et al., Cell, Vol. 79, pp. 315-328 (1994); ENDOSTATIN, described by O'Reilly et al., Cell, Vol. 88, pp. 277-285 (1997); anthranilic acid amides; ZD4190; ZD6474; SU5416; SU6668; bevacizumab; or anti-VEGF antibodies or anti-VEGF receptor antibodies, e.g. rhuMAb and RHUFab, VEGF aptamer e.g. Macugon; FLT-4 inhibitors, FLT-3 inhibitors, VEGFR-2 IgG1 antibody, Angiozyme (RPI 4610) and Bevacizumab (Avastin™).

"Photodynamic therapy", as used herein, refers to therapy which uses certain chemicals known as photosensitizing compounds to treat or prevent cancers. Examples of photodynamic therapy includes treatment with compounds, such as; e.g., VISUDYNE and porfimer sodium.

"Angiostatic steroids", as used herein, refers to compounds which block or inhibit angiogenesis, such as, e.g., anecortave, triamcinolone, hydrocortisone, 11-α-epihydrocotisol, cortexolone, 17α-hydroxyprogesterone, corticosterone, desoxycorticosterone, testosterone, estrone and dexamethasone.

Implants containing corticosteroids refers to compounds, such as, e.g., fluocinolone, dexamethasone.

Other chemotherapeutic compounds include, but are not limited to, plant alkaloids, hormonal compounds and antagonists; biological response modifiers, preferably lymphokines or interferons; antisense oligonucleotides or oligonucleotide derivatives; shRNA or siRNA; or miscellaneous compounds or compounds with other or unknown mechanism of action.

The compounds of the invention are also useful as co-therapeutic compounds for use in combination with other drug substances, such as anti-inflammatory, bronchodilatory or antihistamine drug substances, particularly in the treatment of obstructive or inflammatory airways diseases such as those mentioned hereinbefore, e.g., as potentiators of therapeutic activity of such drugs or as a means of reducing required dosaging or potential side effects of such drugs. A compound of the invention may be mixed with the other drug substance in a fixed pharmaceutical composition or it may be administered separately, before, simultaneously with or after the other drug substance. Accordingly the invention includes a combination of a compound of the invention as hereinbefore described with an anti-inflammatory, bronchodilatory, antihistamine or anti-tussive drug substance, said compound of the invention and said drug substance being in the same or different pharmaceutical composition.

Suitable anti-inflammatory drugs include steroids, in particular, glucocorticosteroids, such as budesonide, beclamethasone dipropionate, fluticasone propionate, ciclesonide or mometasone furoate, or steroids described in WO 02/88167, WO 02/12266. WO 02/100879, WO 02/00679 (especially those of Examples 3, 11, 14, 17, 19, 26, 34, 37, 39, 51, 60, 67, 72, 73, 90, 99 and 101), WO 03/035668, WO 03/048181, WO 03/062259, WO 03/064445, WO 03/072592, non-steroidal glucocorticoid receptor agonists such as those described in WO 00/00531, WO 02/10143, WO 03/082280, WO 03/082787, WO 03/104195, WO 04/005229;

LTB4 antagonists, such as LY293111, CGS025019C, CP-195543, SC-53228, BIIL 284, ONO 4057, SB 209247 and those described in U.S. Patent No. 5,451,100; LTD4 antagonists, such as montelukast and zafirlukast; PDE4 inhibitors, such as cilomilast (Ariflo® GlaxoSmithKline), Roflumilast (Byk Gulden),V-11294A (Napp), BAY19-8004 (Bayer), SCH-351591 (Schering-Plough), Arofylline (Almirall Prodesfarma), PD189659 / PD168787 (ParkeDavis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WHO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; A2a agonists such as those disclosed in EP 409 595 A2, EP 1 052 264, EP 1 241 176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/039762, WO 04/039766, WO 04/045618 and WO 04/046083; A2b antagonists, such as those described in WO 02/42298; and beta-2 adrenoceptor agonists, such as albuterol (salbutamol), metaproterenol, terbutaline, salmeterol fenoterol, procaterol, and especially, formoterol and pharmaceutically acceptable salts thereof, and compounds (in free or salt or solvate form) of formula (I) of WO 00/75114, preferably compounds of the Examples thereof, especially a compound of formula 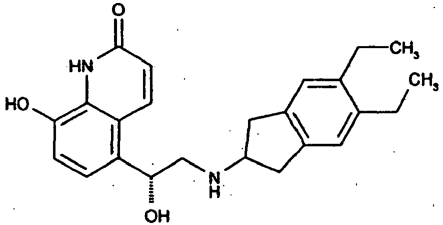 and pharmaceutically acceptable salts thereof, as well as compounds (in free or salt or solvate form) of formula (I) of WO 04/16601, and also compounds of WO 04/033412.

Suitable bronchodilatory drugs include anticholinergic or antimuscarinic compounds, in particular ipratropium bromide, oxitropium bromide, tiotropium salts and CHF 4226 (Chiesi), and glycopyrrolate, but also those described in WO 01/04118, WO 02/5,1841, WO 02/535CA, WO 03/00840, WO 03/87094, WO 04/05285, WO 02/00652, WO 03/53966, EP 424 021, U.S. Patient. No. 5,171,744, U.S. Patent No. 3.714,357, WO 03/33495 and WO 04/018422.

Suitable antihistamine drug substances include cetirizine hydrochloride, acetaminophen, clemastine fumarate, promethazine, loratidine, destoratidine, diphenhydramine and fexofenadine hydrochloride, activastine, astemizole, azelastine, ebastine, epinastine, mizolastine and tefenadine, as well as those disclosed in WO 03/099807, WO 04/026841 and JP 2004107299.

Other useful combinations of compounds of the invention with anti-inflammatory drugs are those with antagonists of chemokine receptors, e.g., CCR-1, CCR-2, CCR-3, CCR-4, CCR-5, CCR-6, CCR-7, CCR-8, CCR-9 and CCR10, CXCR1, CXCR2, CXCR3, CXCR4. CXCR5, particularly CCR-5 antagonists, such as Schering-Plough antagonists SC-351125, SCH-55700 and SCH-D, Takeda antagonists, such as N-[[4-[[[6,7-dihydro-2-(4-methylphenyl)-5H-benzo-cyclohepten-8-yl]carbonyl]amino]phenyl]-methyl]tetrahydro-N,N-dimethyl-2H-pyran-4-aminium chloride (TAK-770), and CCR-5 antagonists described in U.S. Patent No. 6,166,037 (particularly claims 18 and 19), WO 00/66558 (particularly claim 8), WO 00/66559 (particularly claim 9), WO 04/018425 and WO 04/026873.

The structure of the active compounds identified by code nos., generic or trade names may be taken from the actual edition of the standard compendium "The Merck Index" or from databases, e.g., Patents International, e.g., IMS World Publications.

The above-mentioned compounds, which can be used in combination with a compound of the formula (I), can be prepared and administered as described in the art, such as in the documents cited above.

A compound of the formula (I) may also be used to advantage in combination with known therapeutic processes, e.g., the administration of hormones or especially radiation.

A compound of formula (I) may in particular be used as a radiosensitizer, especially for the treatment of tumors which exhibit poor sensitivity to radiotherapy.

By "combination", there is meant either a fixed combination in one dosage unit form, or a kit of parts for the combined administration where a compound of the formula (I) and a combination partner may be administered independently at the same time or separately within time intervals that especially allow that the combination partners show a cooperative; e.g., synergistic effect.

The treatment of non-cancerous, benign brain tumors, especially astrocytomas with the above combination may be a so-called first line treatment, i.e., the treatment of a freshly-diagnosed disease without any preceeding chemotherapy or the like, or it may also be a so-called second line treatment, i.e., the treatment of the disease after a preceeding treatment with imatinib or a BENZIMIDAZOLE DERIVATIVES, depending on the seventy or stage of the disease, as well as the over all condition of the patient, etc.

### Results:

The compound 1-methyl-5-[2-(5-trifluoromethyl-1H-imidazol-2-yl)-pyridin-4-yloxy]-1H-benzoimidazol-2-yl}-(4-trifluoromethyl-phenyl)-amine exhibited potent inhibition, (IC₅₀ <0.1 µM) of B-Raf, c-Raf and mutant B-Raf (V600E) activity as shown below in Table 1.

**Table 1 In Vitro Potency of the Compound 1-Methyl-5-[2-(5-trifluoromethyl-1H-imidazol-2-yl)-pyridin-4-yloxy]-1H-benzoimidazol-2-yl}-(4-trifluoromethyl-phenyl)-amine Against Raf Activity**

| **Target** | **Compound of Example 1 Biochemical IC₅₀** |
|---|---|
| B-Raf (V600E) | 0.0053 µM |
| B-Raf | 0.068 µM |
| c-Raf | 0.004 µM |

As shown above in Table 1, the compound 1-methyl-5-[2-(5-trifluoromethyl-1H-imidazol-2-yl)-pyridin-4-yloxy]-1H-benzoimidazol-2-yl}-(4-trifluoromethyl-phenyl)-amine displays potent inhibitory activity against wild-type isoform B-Raf, wild-type isoform c-Raf, and mutant B-Raf (V600E) Raf kinase. The Raf kinases are activated by Ras and phosphorylate and activate Mek1 and Mek2, which in turn activate Mitogen Activated Kinases 1 and 2 (MAPK), in the MAPK pathway. Raf kinases are known to influence and regulate cellular proliferation, differentiation, survival, oncogenic transformation and apoptosis. The B-Raf isoform has been shown to be the most active form of Raf involved in signaling and key in propagating Ras signaling. The cell-based World Health Organization (WHO) grade 1 astrocytomas including pediatric astrocytomas (Pilomyxoid astrocytoma, pilocytic astrocytomas, pleomorphic xanthoastrocytomas, subependymal giant cell astrocytomas, and subependymomas) are uncommon tumors which can often be cured by surgically removing the tumor (resection). Even if the surgeon is not able to remove the entire tumor, it may remain inactive or be successfully treated with radiation activity of RAF265 in a cell-based activity against B-RafV600E.

As shown below in Table 2, the compound 1-methyl-5-[2-(5-trifluoromethyl-1H-imidazol-2-yl)-pyridin-4-yloxy]-1H-benzoimidazol-2-yl}-(4-trifluoromethyl-phenyl)-amine is a potent inhibitor of VEGFR-2, c-Kit, PDGFR-β and CSF-1R.

**Table 2 Inhibition of Tyrosine Kinases with the Compound 1-Methyl-5-[2-(5-trifluoromethyl-1H-imidazol-2-yl)-pyridin-4-yloxy]-1H-benzoimidazol-2-yl}-(4-trifluoromethyl-phenyl)-amine**

| **Target** | **Compound of Example 1 Biochemical IC₅₀** | **Compound of Example 1 Cell-based EC50** |
|---|---|---|
| VEGF-2 | 0.07 µM | 0.14 µM |
| c-Kit | 0.02 µM | 1.1 µM |
| PDGFR-β | 0.0032 µM | 0.7 µM |
| CSF-1 R | 0.20 µM | ND |

Cell-based assays were also used to measure the activity of the compound 1-methyl-5-[2-(5-trifluoromethyl-1H-imidazol-2-yl)-pyridin-4-yloxy]-1H-benzoimidazol-2-yl}-(4-trifluoromethylphenyl)-amine against the target molecules shown in Table 2 as follows.

Target modulation in HEK-KDR-93 cells after treatment with the compound 1-methyl-5-[2-(5-trifluoromethyl-1H-imidazol-2-yl)-pyridin-4-yloxy]-1H-benzoimidazol-2-yl}-(4-trifluoromethylphenyl)-amine showed inhibition of VEGF mediated VEGFR-2 phosphorylation with an EC₅₀ of 0.19 µM, as measured by a decrease in phospho-VEGFR by ELISA (not shown).

Analysis of inhibition of c-Kit in Mo7e cells after treatment with compound 1-methyl-5-[2-(5-trifluoromethyl-1H-imidazol-2-yl)-pyridin-4-yloxy]-1H-benzoimidazol-2-yl}-(4-trifluoromethylphenyl)-amine showed inhibition of c-Kit phosphorylation with an EC₅₀ of 1.1 µM as measured by a decrease in phospho-c-Kit by ELISA.

Analysis of inhibition of PDGFR-β in MG63 cells after treatment with compound 1-methyl-5-[2-(5-trifluoromethyl-1H-imidazol-2-yl)-pyridin-4-yloxy]-1H-benzoimidazol-2-yl}-(4-trifluoromethyl-phenyl)-amine showed inhibition of phospho-PDGFR-β with an EC₅₀ of 0.7 µM as measured by a decrease in phospho-PDGFR-β by ELISA.

Pathway inhibition and anti-proliferative activity of RAF265 in ST88 cells:

| **Assay** | **EC₅₀ (µM)** |
|---|---|
| phospho-MEK ELISA | 0.15 |
| phospho-ERK ELISA | 0.185 |
| proliferation | 0.207 |

Due to the inhibition of VEGFR-2, RAF265 also has anti-angiogenic activity Which may also provide a therapeutic benefit in treating neurofibromas. To confirm that RAF265 inhibits the growth of new blood vessels (i.e., angiogenesis) *in vivo,* mice were implanted with Matrigel® containing Chinese hamster ovary cells (CHO) overexpressing VEGF and then treated, mice with a dose range of RAF265 or a vehicle control (days 1 and 4). In this model, VEGF expressed from the CHO cells induces angiogenesis within the Matrigel® plug. Plugs are excised on day 5 and assayed for hemoglobin using Drabkin's reagent, as a measure of the degree of angiogenesis.

As shown in Figure 1, VEGF-CHO cells clearly induced angiogenesis, since Matrigel implanted with cells had a much higher level of hemoglobin compared to Matrigel implanted without VEGF-CHO cells. RAF265 caused a dose-dependent decrease in hemoglobin content, with a maximal suppresssion at 50 mg/kg. These data indicate that RAF265 has anti-angiogenic activity *in vivo* and may provide additional anti-tumor activity in astrocytomas.

## Claims

1. Benzimidazole derivative of formula (I): 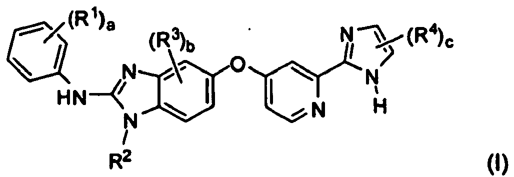 wherein,
each R' is independently selected from hydroxy, halo, C₁₋₆ alkyl, C₁₋₆ alkoxy, (C₁₋₆ alkyl)sulfanyl, (C₁₋₆ alkyl)sulfonyl, cycloalkyl, heterocycloalkyl, phenyl, and heteroaryl; R² is C₁₋₆ alkyl or halo(C₁₋₆ alkyl);
each R³ is independently selected from halo, C₁₋₆ alkyl, and C₁₋₆ alkoxy;
each R⁴ is independently selected from hydroxy, C₁₋₆ alkyl, C₁₋₆ alkoxy, halo, carboxyl, (C₁₋₆ alkoxy)carbonyl, aminocarbonyl, C₁₋₆ alkylaminocarbonyl, carbonitrile, cycloalkyl, heterocycloalkyl, heterocycloalkylcarbonyl, phenyl and heteroaryl;
wherein R¹, R², R³ and R⁴ may be optionally substituted with one or more substituents independently selected from hydroxy, halo, C₁₋₆ alkyl, halo(C₁₋₆ alkyl), C₁₋₆ alkoxy and halo(C₁₋₆ alkoxy);
a is 1, 2, 3, 4, or 5; 1
b is 0, 1, 2, or 3; and
c is 1 or 2;
or a tautomer or stereoisomer thereof or a pharmaceutically acceptable salt of the compound, tautomer or stereoisomer for use in a method of treating astrocytomas.

2. Benzimidazole derivative according to claim 1, where the astrocytomas are selected from Grade I, Grade II, Grade III, or Grade IV astrocytomas.

3. Benzimidazole derivative to claim 1, where the compound of formula (I) is 4-methyl-3-[[4-(3-pyridinyl)-2-pyrimidinyl]amino]-N-[5-(4-methyl-1H-imidazol-1-yl)-3-(trifluoromethyl)phenyl] benzamide of formula (II): 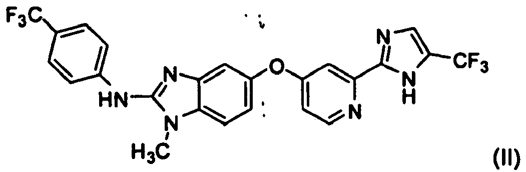 or a tautomer of the compound of formula (II) or a pharmaceutically acceptable salt of the tautomer having the formula (III):

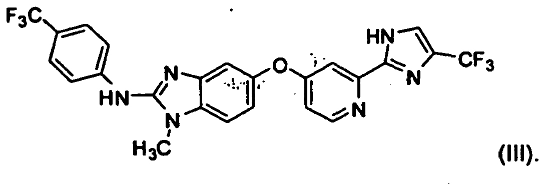

4. Use of a compound of formula (I) 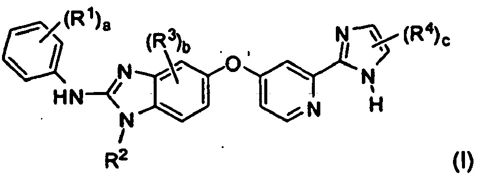 wherein,
each R' is independently selected from hydroxy, halo, C₁₋₆ alkyl, C₁₋₆ alkoxy, (C₁₋₆ alkyl)sulfanyl, (C₁₋₆ alkyl)sulfonyl, cycloalkyl, heterocycloalkyl, phenyl and heteroaryl;
R² is C₁₋₆ alkyl or halo(C₁₋₆ alkyl);
each R³ is independently selected from halo, C₁₋₆ alkyl and C₁₋₆ alkoxy;
each R⁴ is independently selected from hydroxy, C₁₋₆ alkyl, C₁₋₆ alkoxy, halo, carboxyl, (C₁₋₆ alkoxy)carbonyl, aminocarbonyl, C₁₋₆ alkylaminocarbonyl, carbonitrile, cycloalkyl, heterocycloalkyl, heterocycloalkylcarbonyl, phenyl and heteroaryl;
wherein R¹, R², R³, and R⁴ may be optionally substituted with one or more substituents independently selected from hydroxy, halo, C₁₋₆ alkyl, halo(C₁₋₆ alkyl), C₁₋₆ alkoxy and halo(C₁₋₆ alkoxy);
a is 1, 2, 3, 4, or 5;
b is 0, 1, 2, or 3; and
c is 1 or 2;
or a tautomer or stereoisomer, thereof or a pharmaceutically acceptable salt of the compound, tautomer, or stereoisomer for the preparation of a pharmaceutical composition for the treatment of astrocytomas.

5. Use according to Claim 4, wherein said astrocytomas are selected from Grade I, Grade II, Grade III, or Grade IV astrocytomas.

6. The use of a compound of formula (II): 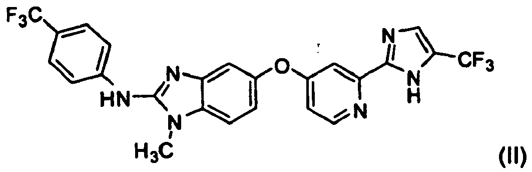 or a tautomer of the compound of formula (II) or a pharmaceutically acceptable salt of the tautomer having the formula (III): 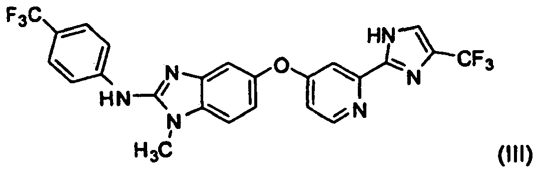 or pharmaceutically acceptable salts thereof for the preparation of a pharmaceutical composition for the treatment of astrocytomas.

7. Use according to Claim 6, where said astrocytomas are selected from Grade I, Grade II, Grade III, or Grade IV astrocytomas.

8. Compound of formula (II): 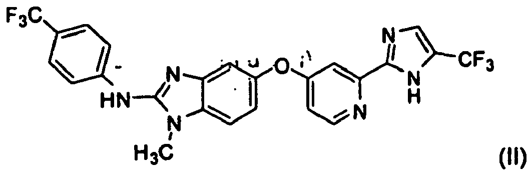 or a tautomer of the compound of formula (II) or a pharmaceutically acceptable salt of the tautomer having the formula (III): 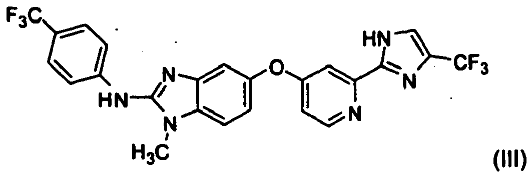 for use in a method for treating mammals, including humans, suffering from astrocytomas.

9. A pharmaceutical preparation for the treatment of astrocytomas comprising a compound of formula (II): 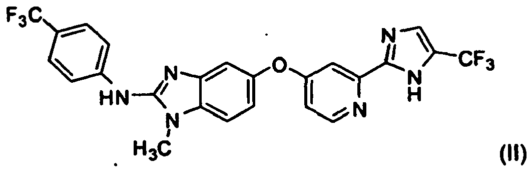 or a tautomer of the compound of formula (II) or a pharmaceutically acceptable salt of the tautomer having the formula (III):

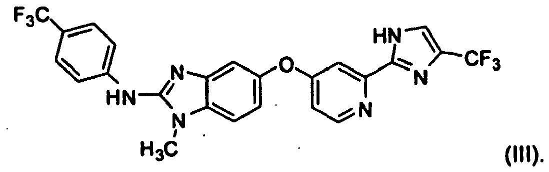

## Patentansprüche

1. Benzimidazolderivat mit der Formel (I): 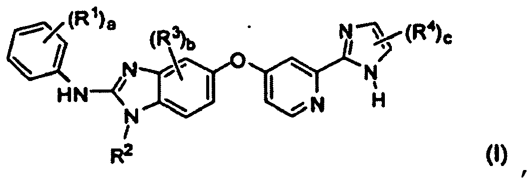 wobei
jedes R¹ unabhängig voneinander ausgewählt ist aus Hydroxy, Halogen, C₁₋₆-Alkyl, C₁₋₆-Alkoxy, (C₁₋₆-Alkyl) sulfanyl, (C₁₋₆-Alkyl)sulfonyl, Cycloalkyl, Heterocycloalkyl, Phenyl und Heteroaryl,
R² C₁₋₆-Alkyl oder Halogen(C₁₋₆-alkyl) ist,
jedes R³ unabhängig voneinander ausgewählt ist aus Halogen, C₁₋₆-Alkyl und C₁₋₆-Alkoxy,
jedes R⁴ unabhängig voneinander ausgewählt ist aus Hydroxy, C₁₋₆-Alkyl, C₁₋₆-Alkoxy, Halogen, Carboxyl, (C₁₋₆-Alkoxy)carbonyl, Aminocarbonyl, C₁₋₆-Alkylaminocarbonyl, Carbonitril, Cycloalkyl, Heterocycloalkyl, Heterocycloalkylcarbonyl, Phenyl und Heteroaryl,
wobei R¹, R², R³ und R⁴ gegebenenfalls mit einem oder mehreren Substituenten, die unabhängig voneinander ausgewählt sind aus Hydroxy, Halogen, C₁₋₆-Alkyl, Halogen (C₁₋₆-alkyl) , C₁₋₆-Alkoxy und Halogen (C₁₋₆-alkoxy), substituiert sein kann oder können,
a 1, 2, 3, 4 oder 5 ist,
b 0, 1, 2 oder 3 ist und
c 1 oder 2 ist,
oder Tautomer oder Stereoisomer davon, oder pharmazeutisch akzeptables Salz der Verbindung, des Tautomers oder Stereoisomers,
zur Verwendung in einem Verfahren zur Behandlung von Astrozytomen.

2. Benzimidazolderivat nach Anspruch 1, wobei die Astrozytome ausgewählt sind aus Grad I-, Grad II-, Grad III- oder Grad IV-Astrozytomen.

3. Benzimidazolderivat nach Anspruch 1, wobei die Verbindung der Formel (I) 4-Methyl-3-[[4-(3-pyridinyl)-2-pyrimidinyl]amino]-N-[5-(4-methyl-1H-imidazol-1-yl)-3-(trifluormethyl)phenyl]benzamid der Formel (II) ist: 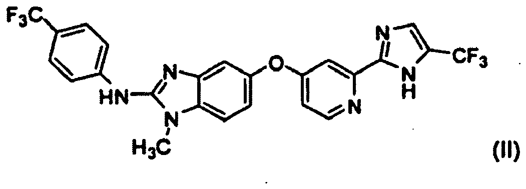 oder Tautomer der Verbindung mit der Formel (II) oder pharmazeutisch akzeptables Salz des Tautomers mit der Formel (III):

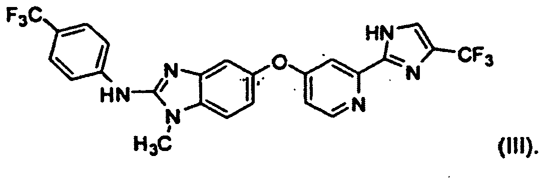

4. Verwendung einer Verbindung mit der Formel (I) 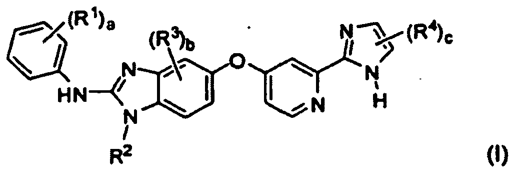 wobei
jedes R¹ unabhängig voneinander ausgewählt ist aus Hydroxy, Halogen, C₁₋₆-Alkyl, C₁₋₆-Alkoxy, (C₁₋₆-Alkyl) sulfanyl, (C₁₋₆-Alkyl)sulfonyl, Cycloalkyl, Heterocycloalkyl, Phenyl und Heteroaryl,
R² C₁₋₆-Alkyl oder Halogen (C₁₋₆-alkyl) ist,
jedes R³ unabhängig voneinander ausgewählt ist aus Halogen, C₁₋₆-Alkyl und C₁₋₆-Alkoxy,
jedes R⁴ unabhängig voneinander ausgewählt ist aus Hydroxy, C₁₋₆-Alkyl, C₁₋₆-Alkoxy, Halogen, Carboxyl, (C₁₋₆-Alkoxy) carbonyl, Aminocarbonyl, C₁₋₆-Alkylaminocarbonyl, Carbonitril, Cycloalkyl, Heterocycloalkyl, Heterocycloalkylcarbonyl, Phenyl und Heteroaryl,
wobei R¹, R², R³ und R⁴ gegebenenfalls mit einem oder mehreren Substituenten, die unabhängig voneinander ausgewählt sind aus Hydroxy, Halogen, C₁₋₆-Alkyl, Halogen (C₁₋₆-alkyl) , C₁₋₆-Alkoxy und Halogen(C₁₋₆-alkoxy), substituiert sein kann oder können,
a 1, 2, 3, 4 oder 5 ist,
b 0, 1, 2 oder 3 ist und
c 1 oder 2 ist,
oder Tautomer oder Stereoisomer davon, oder pharmazeutisch akzeptables Salz der Verbindung, des Tautomers oder Stereoisomers,
zur Herstellung einer pharmazeutischen Zusammensetzung zur Behandlung von Astrozytomen.

5. Verwendung nach Anspruch 4, wobei die Astrozytome ausgewählt sind aus Grad I-, Grad II-, Grad III- oder Grad IV-Astrozytomen.

6. Verwendung einer Verbindung mit der Formel (II) : 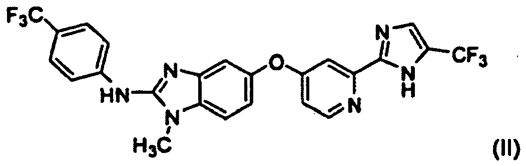 oder Tautomer der Verbindung mit der Formel (II) oder pharmazeutisch akzeptables Salz des Tautomers mit der Formel (III): 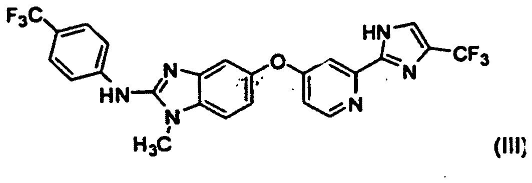 oder pharmazeutisch akzeptables Salz davon,
zur Herstellung einer pharmazeutischen Zusammensetzung zur Behandlung von Astrozytomen.

7. Verwendung nach Anspruch 6, wobei die Astrozytome ausgewählt sind aus Grad I-, Grad II-, Grad III- oder Grad IV-Astrozytomen.

8. Verbindung mit der Formel (II): 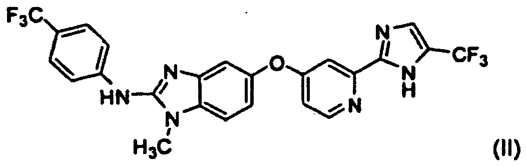 oder Tautomer der Verbindung mit der Formel (II) oder pharmazeutisch akzeptables Salz des Tautomers mit der Formel (III) : 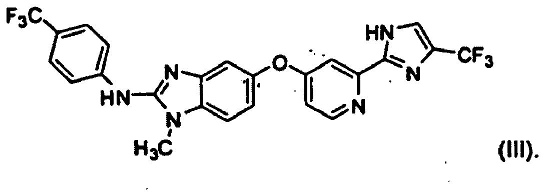 zur Verwendung in einem Verfahren zur Behandlung von Säugetieren, einschließlich Menschen, die unter Astrozytomen leiden.

9. Pharmazeutische Zubereitung zur Behandlung von Astrozytomen, umfassend eine Verbindung der Formel (II): 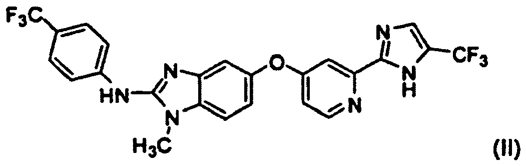 oder Tautomer der Verbindung mit der Formel (II) oder pharmazeutisch akzeptables Salz des Tautomers mit der Formel (III):

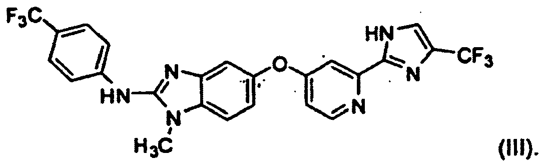

## Revendications

1. Dérivé de benzimidazole de formule (I): 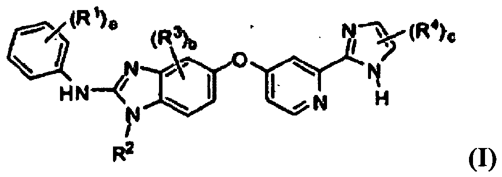 dans laquelle
chaque R¹ est indépendamment choisi parmi un groupe hydroxy, halogéno, alkyle en C₁₋₆, alcoxy en C₁₋₆, alkyl(en C₁₋₆)sulfanyle, alkyl(en C₁₋₆)sulfonyle, cycloalkyle, hétérocycloalkyle, phényle et hétéroaryle;
R² est un groupe alkyle en C₁₋₆ ou halogéno(alkyle en C₁₋₆);
chaque R³ est indépendamment choisi parmi un groupe halogéno, alkyle en C₁₋₆, et alcoxy en C₁₋₆;
chaque R⁴ est indépendamment choisi parmi un groupe hydroxy, alkyle en C₁₋₆, alcoxy en C₁₋₆, halogéno, carboxyle, alcoxy(en C₁₋₆)carbonyle, aminocarbonyle, alkyl(en C₁₋₆)aminocarbonyle, carbonitrile, cycloalkyle, hétérocycloalkyle, hétérocycloalkylcarbonyle, phényle et hétéroaryle;
dans laquelle R¹, R², R³ et R⁴ peuvent être facultativement substitués par un ou plusieurs substituants indépendamment choisis parmi les substituants hydroxy, halogéno, alkyle en C₁₋₆, halogéno(alkyle en C₁₋₆), alcoxy en C₁₋₆ et halogéno(alcoxy en C₁₋₆);
a est égal à 1, 2, 3, 4 ou 5;
b est égal à 0, 1, 2 ou 3; et
c est égal à 1 ou 2;
ou l'un de ses tautomères ou stéréo-isomères ou un sel pharmaceutiquement acceptable du composé, du tautomère ou du stéréo-isomère, à utiliser dans un procédé de traitement des astrocytomes.

2. Dérivé de benzimidazole selon la revendication 1, où les astrocytomes sont choisis parmi les astrocytomes de Grade I, de Grade II, de Grade III ou de Grade IV.

3. Dérivé de benzimidazole selon la revendication 1, où le composé de formule (I) est le 4-méthyl-3-[[4-(3-pyridinyl)-2-pyrimidinyl]amino]-N-[5-(4-méthyl-1H-imidazol-1-yl)-3-(trifluorométhyl)phényl]benzamide de formule (II): 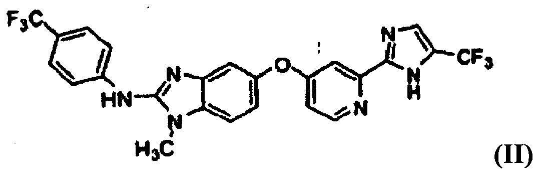 ou un tautomère du composé de formule (II) ou un sel pharmaceutiquement acceptable du tautomère répondant à la formule (III):

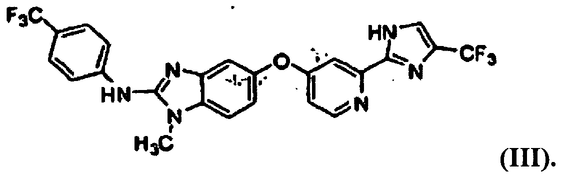

4. Utilisation d'un composé de formule (I) 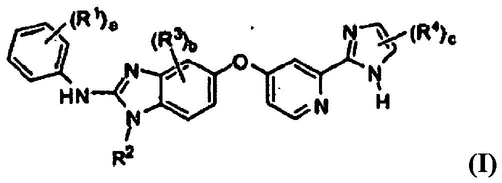 dans laquelle
chaque R¹ est indépendamment choisi parmi un groupe hydroxy, halogéno, alkyle en C₁₋₆, alcoxy en C₁₋₆, alkyl(en C₁₋₆)sulfanyle, alkyl(en C₁₋₆)sulfonyle, cycloalkyle, hétérocycloalkyle, phényle et hétéroaryle;
R² est un groupe alkyle en C₁₋₆ ou halogéno(alkyle en C₁₋₆);
chaque R³ est indépendamment choisi parmi un groupe halogéno, alkyle en C₁₋₆, et alcoxy en C₁₋₆;
chaque R⁴ est indépendamment choisi parmi un groupe hydroxy, alkyle en C₁₋₆, alcoxy en C₁₋₆, halogéno, carboxyle, alcoxy(en C₁₋₆)carbonyle, aminocarbonyle, alkyl(en C₁₋₆)aminocarbonyle, carbonitrile, cycloalkyle, hétérocycloalkyle, hétérocycloalkylcarbonyle, phényle et hétéroaryle;
dans laquelle R¹, R², R³ et R⁴ peuvent être facultativement substitués par un ou plusieurs substituants indépendamment choisis parmi les substituants hydroxy, halogéno, alkyle en C₁₋₆, halogéno(alkyle en C₁₋₆), alcoxy en C₁₋₆ et halogéno(alcoxy en C₁₋₆);
a est égal à 1, 2, 3, 4 ou 5;
b est égal à 0, 1, 2 ou 3; et
c est égal à 1 ou 2;
ou de l'un de ses tautomères ou stéréo-isomères, ou d'un sel pharmaceutiquement acceptable du composé, du tautomère ou du stéréo-isomère, pour la préparation d'une composition pharmaceutique destinée au traitement des astrocytomes.

5. Utilisation selon la revendication 4, où lesdits astrocytomes sont choisis parmi les astrocytomes de Grade I, de Grade II, de Grade III ou de Grade IV.

6. Utilisation d'un composé de formule (II): 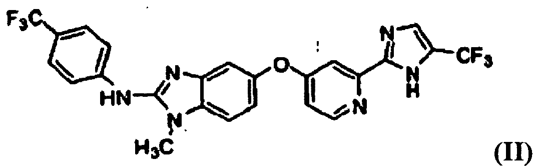 ou d'un tautomère du composé de formule (II) ou d'un sel pharmaceutiquement acceptable du tautomère répondant à la formule (III): 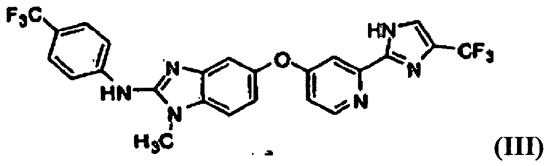 ou de leurs sels pharmaceutiquement acceptables pour la préparation d'une composition pharmaceutique destinée au traitement des astrocytomes.

7. Utilisation selon la revendication 6, où lesdits astrocytomes sont choisis parmi les astrocytomes de Grade I, de Grade II, de Grade III ou de Grade IV.

8. Composé de formule (II): 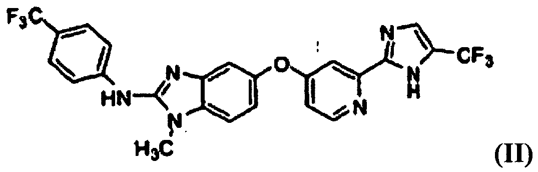 ou tautomère du composé de formule (II) ou sel pharmaceutiquement acceptable du tautomère répondant à la formule (III): 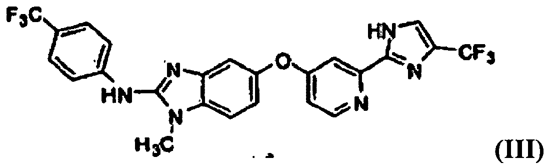 à utiliser dans un procédé destiné au traitement des mammifères, notamment les humains, souffrant d'astrocytomes.

9. Préparation pharmaceutique destinée au traitement des astrocytomes, comprenant un composé de formule (II): 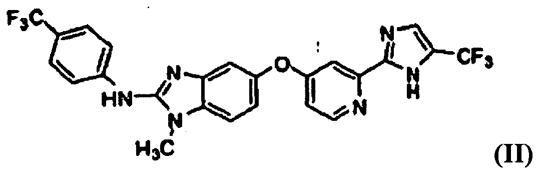 ou un tautomère du composé de formule (II) ou un sel pharmaceutiquement acceptable du tautomère répondant à la formule (III):
